(19) Europäisches Patentamt
European Patent Office
Office européen des brevets

(11) EP 0 659 069 B1

(12) **FASCICULE DE BREVET EUROPEEN**

(45) Date de publication et mention de la délivrance du brevet:
**20.11.1996 Bulletin 1996/47**

(21) Numéro de dépôt: **93919448.6**

(22) Date de dépôt: **13.09.1993**

(51) Int. Cl.$^6$: **A61K 7/48**, A61K 7/00, C09B 67/00

(86) Numéro de dépôt international:
**PCT/FR93/00878**

(87) Numéro de publication internationale:
**WO 94/06406 (31.03.1994 Gazette 1994/08)**

# (54) COMPOSITION COSMETIQUE CONTENANT DES PARTICULES SOLIDES ORGANIQUES REVETUES D'UN POLYMERE CATIONIQUE

KOSMETISCHE ZUSAMMENSETZUNG, DIE FESTE ORGANISCHE, MIT EINEM KATIONISCHEN POLYMER ÜBERZOGENE PARTIKEL ENTHÄLT

COSMETIC COMPOSITION CONTAINING SOLID ORGANIC PARTICLES COATED WITH A CATIONIC POLYMER

(84) Etats contractants désignés:
**AT BE CH DE DK ES FR GB GR IE IT LI NL PT SE**

(30) Priorité: **11.09.1992 FR 9210885**

(43) Date de publication de la demande:
**28.06.1995 Bulletin 1995/26**

(73) Titulaire: **L'OREAL**
**75008 Paris (FR)**

(72) Inventeurs:
- **MELLUL, Myriam**
  **F-94240 L'Hay-les-Roses (FR)**
- **CANDAU, Didier**
  **F-77000 Melun (FR)**

(74) Mandataire: **Tonnellier, Jean-Claude**
**Cabinet Nony & Cie.**
**29, rue Cambacérès**
**75008 Paris (FR)**

(56) Documents cités:
**EP-A- 0 209 879**
**EP-A- 0 212 870**
**EP-A- 0 220 617**
**EP-A- 0 445 342**
**FR-A- 2 234 359**
**GB-A- 2 107 186**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.13.8/3.4

## Description

La présente invention a pour objet des compositions cosmétiques, contenant une dispersion de particules solides, dans lesquelles sont introduites des particules organiques solides dont la surface est revêtue à l'aide d'un polymère cationique.

On sait que divers produits de maquillage tels que les poudres libres ou compactées, les fonds de teint, les fards à joues, les fards à paupières, ainsi que les rouges à lèvres sont présentés sous la forme de compositions comprenant une dispersion de particules organiques solides dans un liant gras. Il peut s'agir de compositions anhydres, ou bien d'émulsions huile-dans-l'eau ou eau-dans-l'huile.

Selon les types de compositions, les particules organiques solides sont notamment des pigments (blancs et/ou colorés), destinés à conférer à la peau du visage ou des lèvres une certaine coloration, ou même à colorer les compositions elles-mêmes, ou encore des particules jouant le rôle de charges (en particulier dans les compositions sous forme de poudres).

Dans les rouges à lèvres, les particules solides dispersées dans un liant gras approprié sont surtout des pigments colorés, éventuellement en association avec des pigments blancs (par exemple de fines particules de dioxyde de titane) qui permettent de nuancer les teintes apportées par les pigments colorés.

On utilise également de tels pigments colorés dans les compositions de vernis à ongles, qui sont essentiellement constituées d'une dispersion de ces pigments dans une solution d'un polymère filmogène et d'un plastifiant dans un solvant organique approprié.

La préparation et l'utilisation des compositions cosmétiques contenant des dispersions de particules solides posent plusieurs sortes de problèmes. Un problème commun a la réalisation de l'ensemble des compositions dont on vient de parler réside dans la difficulté d'obtenir des dispersions stables, de façon à appliquer, par exemple sur la peau, un maquillage régulier dont l'application est uniforme et qui conserve une bonne homogénéité. Pour cela, les spécialistes ont été amenés à effectuer des traitements de surface sur les poudres utilisées, notamment afin de modifier les propriétés interfaciales intervenant dans les phénomènes de mouillage et de dispersion. Le but de ces traitements est souvent de rendre la poudre hydrophobe afin de favoriser son incorporation dans les liants et les huiles de formulation, et d'augmenter la stabilité de la dispersion en diminuant les phénomènes de floculation et d'agrégation ; voir par exemple le brevet européen 279 319 qui décrit l'enrobage des pigments par des polymères siliconés.

Ces traitements permettent donc de régler les problèmes de stabilité de la dispersion, en limitant les phénomènes de floculation. Toutefois, ils ne règlent pas un autre problème important, à savoir les faibles propriétés d'adhérence sur la peau des particules solides. En effet, on sait que les particules solides utilisées notamment dans les compositions sous forme de poudres, n'ont que de faibles propriétés d'adhérence sur la peau. Les traitements de surface destinés à améliorer la stabilité des dispersions dans les liants gras n'apportent pas d'amélioration sensible en ce qui concerne les propriétés d'adhérence.

On sait par ailleurs que les produits de maquillage pour le visage et pour les yeux sont souvent présentés sous la forme de poudres compactées. Les poudres compactées sont préparées par mélange des constituants de la poudre avec un agent liant, puis mise sous la forme désirée par compression dans des conteneurs appropriés.

Les poudres compactées doivent présenter des caractéristiques de dureté particulières. La dureté est fonction de la pression de compactage appliquée. Si le produit compacté est trop mou, il sera très fragile et une quantité trop importante de produit sera prélevée au moment de l'application. Par contre, s'il est trop dur, le délitage sera difficile. Par ailleurs, un produit compact doit présenter une surface parfaitement plane. Enfin, il doit répondre favorablement au test de chute, c'est-à-dire présenter une perte de poids réduite après une chute effectué dans des conditions normalisées.

On a maintenant découvert qu'il est possible d'obtenir des compositions cosmétiques comprenant une dispersion de pigments organiques solides dans un liant, ayant de bonnes propriétés de stabilité et d'adhérence sur la peau ou sur les phanères, en introduisant dans lesdites compositions des particules organiques solides dont la surface a été revêtue avec un polymère cationique. On a constaté, de façon surprenante, que le revêtement des particules organiques solides par des polymères cationiques, qui constituent pourtant un revêtement hydrophile, n'empêche pas l'obtention d'une bonne dispersibilité des particules dans les liants gras. En outre, les compositions compactées obtenues avec des particules organiques solides revêtues de polymère cationique présentent de façon inattendue de bonnes propriétés de cohésion qui se traduisent notamment par un comportement très satisfaisant dans les tests de chute. Par ailleurs, les compositions ainsi obtenues ont de bonnes propriétés d'adhérence sur la peau ou sur les phanères après application.

La présente invention a donc pour objet une composition cosmétique pour la peau ou les phanères, comprenant une dispersion de particules organiques solides dans un liant, caractérisée par le fait qu'au moins une partie desdites particules organiques sont introduites dans ladite composition sous la forme de particules dont la surface est revêtue d'au moins un polymère cationique.

Dans les compositions de l'invention, des particules organiques solides sont revêtues avec le polymère cationique en surface. Cela signifie qu'après revêtement, il n'y a ni changement de morphologie ni modification notable des tailles des particules, comme on peut le vérifier par microscopie électronique.

Dans la présente demande, l'expression "polymère cationique" désigne un polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne polymère soit être portés par un substituant latéral.

Les polymères cationiques utilisés ont de préférence une masse moléculaire comprise entre $10^3$ et $3.10^6$ environ.

De préférence, les particules organiques revêtues utilisées dans les compositions de l'invention sont revêtues uniquement avec un (ou plusieurs) polymère(s) cationiques(s).

Les polymères cationiques utilisés sont en particulier ceux ayant un taux de quaternisation, exprimé en équivalents cationiques par gramme de polymère, d'au moins 0,05 méq cationique/g. On utilise notamment des polymères cationiques qui contiennent par exemple au moins 10% en poids de motifs comportant des groupements amine ou ammonium quaternaire.

Lorsque le polymère cationique contient des groupements amine ou ammonium quaternaire portés par un substituant latéral, la chaîne polymère est par exemple une chaîne acrylique, vinylique, siliconée, fluorée ou saccharidique.

Parmi les polymères cationiques, on peut citer plus particulièrement les protéines (ou hydrolysats de protéines) quaternisées, les polysiloxanes quaternisés et les polymères du type polyamine, polyaminoamide et polyammonium quaternaire. Ce sont des produits connus.

De préférence, on utilise des polymères cationiques ne contenant pas de silicium, c'est-à-dire autres que des polymères siliconés.

On préfère utiliser plus particulièrement comme polymère cationique un polymère à groupement(s) amine primaire, secondaire, ou tertiaire ionisé(s) ou à groupement(s) ammonium quaternaire, ces derniers étant préférés.

Le groupement ammonium quaternaire peut être obtenu notamment par quaternisation de groupements aminés avec des agents de quaternisation classiques tels que des halogénures d'alkyle ou d'aralkyle (par exemple iodure de méthyle, bromure d'éthyle, chlorure de benzyle), des sulfates d'alkyle (par exemple sulfate de diméthyle), etc...

Les groupements amine ionisés sont obtenus par salification des groupement aminés avec des acides organiques ou minéraux tels que les acides chlorhydrique, bromhydrique, lactique, acétique, glycolique, etc...

Les quantités de polymère déposées sur les particules varient avec le mode opératoire utilisé pour le revêtement. Généralement, la proportion pondérale de polymère cationique, par rapport au poids total des particules revêtues, est au moins égale à 0,1% ; la limite supérieure de la quantité de polymère cationique est suffisamment faible pour que les particules gardent leur individualité et leur forme. Autrement dit, le polymère cationique forme, au plus, une couche mince (éventuellement lacunaire) sur les particules revêtues. Le plus souvent, la proportion pondérale de polymère cationique, dans les particules revêtues, est inférieure à 10% et en particulier inférieure à 8%, par rapport au poids total des particules revêtues.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1500 à 10 000, et en particulier de 2 000 à 5 000 environ. Parmi ces composés, on peut citer notamment :

- les hydrolysats de collagène portant des groupements triéthylammonium tels que les produits vendus sous la dénomination "Quat-Pro E" par la Société Maybrook et dénommés dans le dictionnaire CTFA "Triethonium Hydrolyzed Collagen Ethosulfate";
- les hydrolysats de collagène portant des groupements chlorure de triméthylammonium ou de triméthylstéarylammonium, vendus sous la dénomination de "Quat-Pro Si" par la Société Maybrook et dénommés dans le dictionnaire CTFA "Steartrimonium Hydrolyzed Collagen";
- les hydrolysats de protéines animales portant des groupements triméthylbenzylammonium tels que les produits vendus sous la dénomination "Crotein BTA" par la Société Croda et dénommés dans le dictionnaire CTFA "Benzyltrimonium hydrolyzed animal protein";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :

- le "Croquat L" dont les groupements ammonium quaternaires comportent un groupement alkyle en $C_{12}$;
- le "Croquat M" dont les groupements ammonium quaternaires comportent des groupements alkyle en $C_{10}$-$C_{18}$:
- le "Croquat S" dont les groupements ammonium quaternaires comportent un groupement alkyle en $C_{18}$;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule :

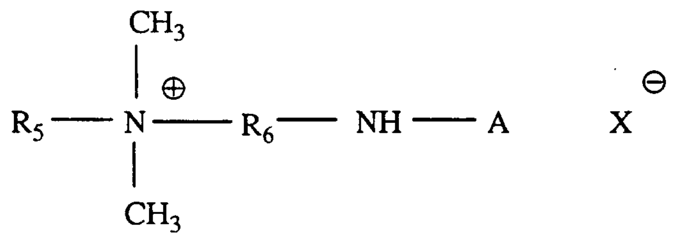

(I)

dans laquelle $X^-$ est un anion d'un acide organique ou minéral, A désigne un reste de protéine dérivé d'hydrolysats de protéine de collagène, $R_5$ désigne un groupement lipophile comportant jusqu'à 30 atomes de carbone, $R_6$ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagen Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Une autre famille de polymères cationiques est celle des polymères cationiques siliconés. Parmi ces polymères, on peut citer :

(a) les polysiloxanes quaternisés dénommés dans le dictionnaire CTFA "Amodiméthicone" et répondant à la formule ;

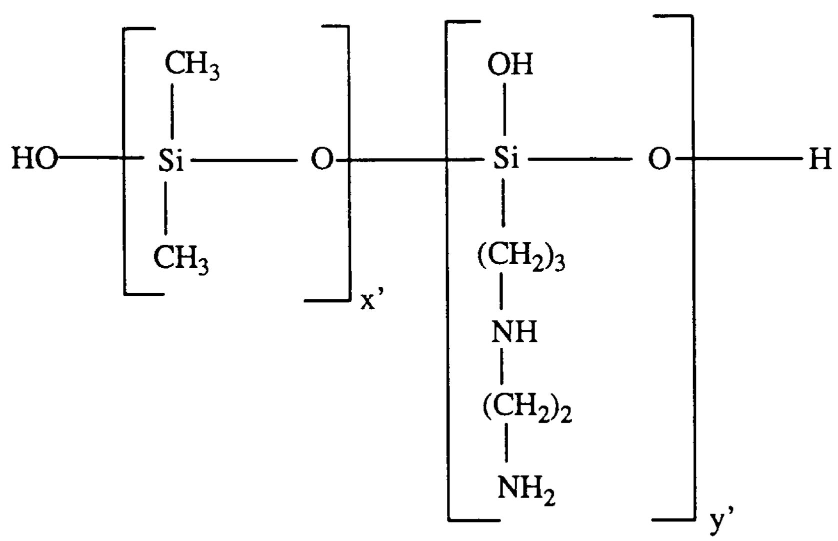

(II)

dans laquelle x' et y' sont des nombres entiers dépendant du poids moléculaire, généralement tels que ledit poids moléculaire est compris entre 5000 et 10000 environ ;

(b) les polymères cationiques siliconés répondant à la formule :

$$R'_aG_{3-a}\text{- }Si(OSiG_2)_n\text{- }(OSiG_bR'_{2-b})_m\text{- }O\text{-}SiG_{3-a}\text{-}R'_a \qquad (III)$$

dans laquelle :

G est un atome d'hydrogène, ou un groupement phényle, OH, ou alkyle en $C_1$-$C_8$, par exemple méthyle,

a désigne le nombre 0 ou un nombre entier de 1 à 3, en particulier O,

b désigne O ou 1, et en particulier 1,

m et n sont des nombres tels que la somme (n+m) peut varier notamment de 1 à 2000 et en particulier de 50 à 150, n pouvant désigner un nombre de 0 à 1999 et notamment de 49 à 149 et m pouvant désigner un nombre de 1 à 2000, et notamment de 1 à 10 ;

R' est un radical monovalent de formule $-C_qH_{2q}L$ dans laquelle q est un nombre de 2 à 8 et L est un groupement aminé éventuellement quaternisé choisi par exemple parmi les groupements :

-NR"-$CH_2$-$CH_2$-N'(R")$_2$
-N(R")$_2$
-$N^{\oplus}$(R")$_3$ $A^{\ominus}$
-$N^{\oplus}$(R")$H_2$ $A^{\ominus}$
-N(R")-$CH_2$-$CH_2$-$N^{\oplus}$R" $H_2$ $A^{\ominus}$,

dans lesquels R" peut désigner hydrogène, phényle, benzyle, ou un radical hydrocarboné saturé monovalent, par exemple un radical alkyle ayant de 1 à 20 atomes de carbone et $A^{\ominus}$ représente un ion halogénure tel que par exemple fluorure, chlorure, bromure ou iodure.

Un produit correspondant à cette définition est le polymère dénommé "triméthylsilylamodiméthicone", répondant à la formule :

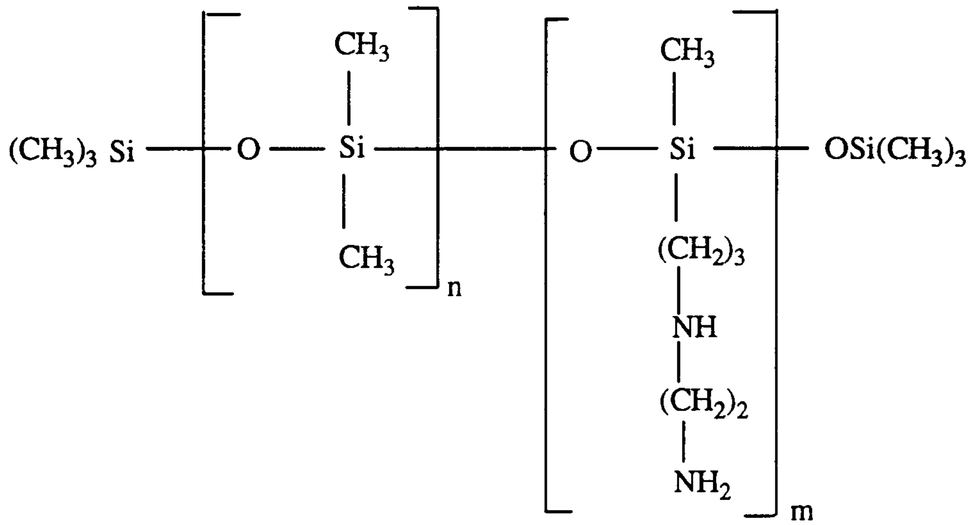

(IV)

dans laquelle n et m ont les significations données ci-dessus (cf. formule III). De tels polymères sont décrits par exemple dans la demande de brevet EP-A-95238.

(c) les polymères cationiques siliconés répondant à la formule :

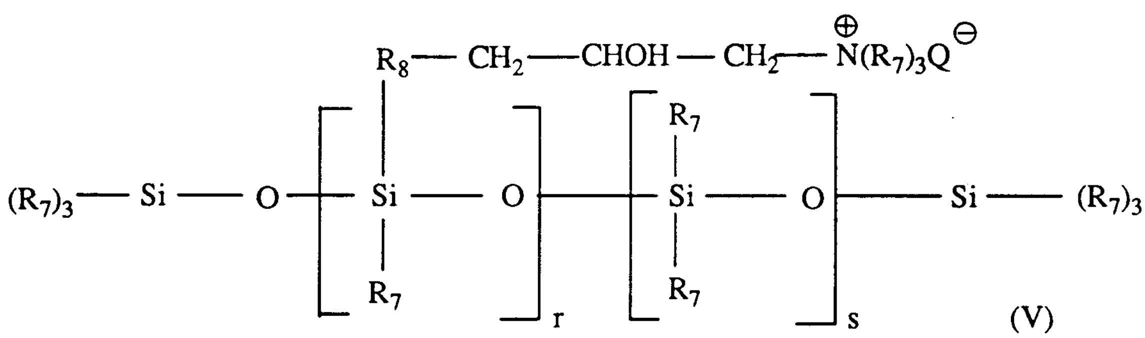

(V)

dans laquelle

$R_7$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;

$R_8$ désigne un radical hydrocarboné divalent, notamment un radical alkylène en $C_1$-$C_{18}$ ou un radical alkylèneoxy divalent en $C_1$-$C_{18}$, par exemple en $C_1$-$C_8$ ;

$Q^-$ est un ion halogénure, notamment chlorure ;

r représente une valeur statistique moyenne de 2 à 20 et en particulier de 2 à 8 ;

s représente une valeur statistique moyenne de 20 à 200 et en particulier de 20 à 50.

De tels polymères sont décrits plus particulièrement dans le brevet US-4 185 087.

Un polymère entrant dans cette classe est le polymère vendu par la Société Union Carbide sous la dénomination "Ucar Silicone ALE 56".

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit vendu sous la dénomination "Emulsion Cationique DC 929" par la Société Dow Corning qui comprend, outre l'amodiméthicone, un agent de surface cationique comprenant un mélange de produits répondant à la formule :

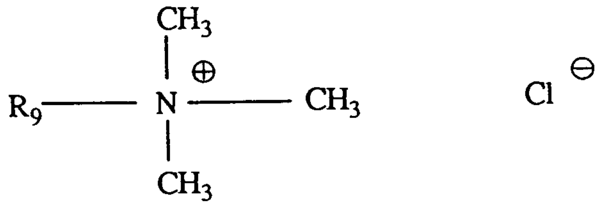

dans lequel $R_9$ désigne des radicaux alcényle et/ou alcoyle ayant de 14 à 22 atomes de carbone, dérivés des acides gras du suif,
en association avec un agent de surface non ionique de formule :

$$C_9H_{19}\text{-}C_6H_4\text{-}(OC_2H_4)_{10}\text{-}OH$$

connu sous la dénomination "Nonoxynol 10".

Un autre produit commercial utilisable selon l'invention est le produit vendu sous la dénomination"Dow Corning Q2 7224" par la Société Dow Corning comportant en association le triméthylsilylamodiméthicone de formule (IV), un agent de surface non ionique de formule :

$$C_8H_{17}\text{-}C_6H_4\text{-}(OCH_2CH_2)_n\text{-}OH \text{ où } n = 40$$

dénommé encore octoxynol-40,

un autre agent de surface non ionique de formule :

$$C_{12}H_{25}\text{-}(OCH_2\text{-}CH_2)_n\text{-}OH \text{ où } n = 6$$

encore dénommé isolaureth-6,

et du glycol.

Les polymères du type polyamine, polyaminoamide, polyammonium quaternaire, utilisables conformément à la présente invention peuvent être notamment ceux mentionnés dans les brevets français n° 2 505 348 ou 2 542 997. Parmi ces polymères, on peut citer :

(1) les copolymères vinylpyrrolidone-acrylate ou -méthacrylate de dialkylaminoalkyle quaternisés ou non, tels que les produits vendus sous la dénomination "Gafquat" par la Société GAF Corporation, comme par exemple Gafquat 734, 755 ou HS100 ou bien le produit dénommé "Copolymère 937". Ces polymères sont décrits en détail dans les brevets français 2 077 143 et 2 393 573.

(2) Les dérivés d'éthers de cellulose comportant des groupements ammonium quaternaires décrits dans le brevet français 1 492 597, et en particulier les polymères commercialisés sous les dénominations "JR" (JR 400; JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US-A 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, ou de diméthyldiallylammonium.

Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US-3 589 578 et 4 031 307, et plus particulièrement le produit commercialisé sous la dénomination "Jaguar C. 13 S" vendu par la Société Meyhall.

(5) Les polymères constitués de motifs pipérazinyle et de radicaux divalents alkylène ou hydroxyalkylène à chaînes droites ou ramifiées, éventuellement interrompues par un ou des atomes d'oxygène, de soufre, d'azote et/ou par des cycles aromatiques ou des hétérocycles, ainsi que les produits d'oxydation et/ou de quaternisation de ces polymères. De tels polymères sont décrits notamment dans les brevets français 2 162 025 et 2 280 361.

(6) Les polyaminopolyamides solubles dans l'eau préparés en particulier par polycondensation d'un composé acide avec une polyamine. Ces polyaminopolyamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alcoyle ou encore par un oligomère résultant de la réaction d'un composé bifonctionnel réactif vis-à-vis d'une bis-halohydrine, d'un bis-azétidinium, d'une bis-haloacryldiamine, d'un bis-halogénure d'alkyle, d'une épihalohydrine, d'un diépoxyde ou d'un dérivé bis-insaturé ; l'agent réticulant étant utilisé notamment dans des proportions allant de 0,025 à 0,35 mole par groupement amine du polyaminopolyamide.

Ces polyaminopolyamides peuvent être alcoylés ou, s'ils comportent une ou plusieurs fonctions amines tertiaires, quaternisés.

De tels polymères sont décrits en particulier dans les brevets français 2 252 840 et 2 368 508.

(7) Les dérivés de polyaminopolyamides résultant de la condensation de polyalcoylène polyamines avec des acides polycarboxyliques suivie d'une alcoylation par des agents bifonctionnels. On peut citer par exemple les polymères acide adipique-dialcoylamino hydroxyalcoyldialcoylène triamine dans lesquels le radical alcoyle comporte de 1 à 4 atomes de carbone et désigne notamment méthyle, éthyle ou propyle. De tels polymères sont décrits dans le brevet français 1 583 363.

Parmi ces dérivés, on peut citer plus particulièrement les polymères acide adipique/diméthylaminohydroxypropyl/ diéthylènetriamine vendus sous les dénominations "Cartarétine F, $F_4$ ou $F_8$" par la Société Sandoz.

(8) Les polymères obtenus par réaction d'une polyalkylène polyamine comportant deux groupements amine primaire et au moins un groupement amine secondaire avec un acide dicarboxylique choisi par exemple parmi l'acide diglycolique et les acides dicarboxyliques aliphatiques saturés ayant de 3 à 8 atomes de carbone, le rapport molaire entre la polyalkylène polyamine et l'acide dicarboxylique étant compris par exemple entre 0,8 : 1 et 1,4 : 1, et le polyaminopolyamide résultant étant ensuite amené à réagir avec l'épichlorhydrine dans un rapport molaire d'épichlorhydrine par rapport aux groupements amine secondaire du polyaminopolyamide compris entre 0,5 : 1 et 1,8 : 1. De tels polymères sont décrits en particulier dans les brevets US-3 227 615 et 2 961 347.

Des polymères de ce type sont en particulier commercialisés sous la dénomination "Hercosett 57" par la Société Hercules Incorporated ou bien sous la dénomination "PD 170" ou "Delsette 101" par la Société Hercules.

(9) Les cyclopolymères tels que des homopolymères comportant comme constituants principaux de la chaîne des motifs répondant aux formules (VI) ou (VI').

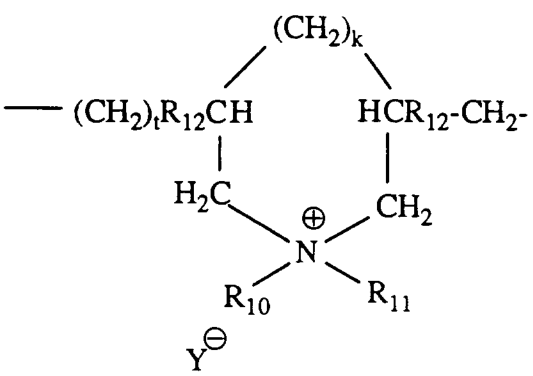

(VI)

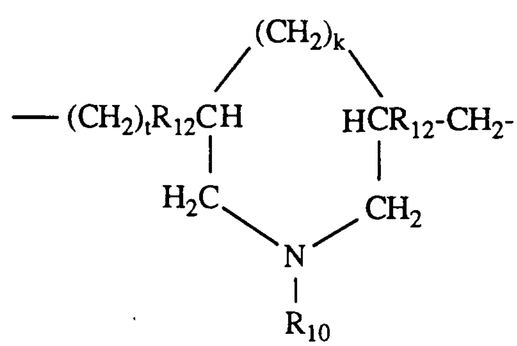

(VI')

dans lesquelles k et t sont égaux à O ou 1, et la somme k + t = 1 , $R_{12}$ désigne hydrogène ou méthyle, $R_{10}$ et $R_{11}$ désignent indépendamment un groupement alcoyle ayant de 1 à 22 atomes de carbone, un groupement hydroxyalcoyle dans lequel le groupement alcoyle a notamment 1 à 5 atomes de carbone, un groupement amidoalcoyle inférieur dont l'alcoyle a notamment 1 à 6C, $R_{10}$ et $R_{11}$ peuvent désigner conjointement avec l'atome d'azote auquel ils sont rattachés des groupements hétérocycliques tels que pipéridinyle ou morpholinyle, ainsi que les copolymères comportant à la fois des motifs de formules (VI) ou (VI') et des motifs dérivés d'acrylamide ou de diacétone acrylamide, $Y^{\ominus}$ est un anion tel que bromure, chlorure, acétate, borate, citrate, tartrate, bisulfate, bisulfite, sulfate, phosphate, etc... Parmi les polymères définis ci-dessus, on peut citer plus particulièrement l'homopolymère de chlorure de diméthyl diallyl ammonium vendu sous la dénomination Merquat 100 et le copolymère de chlorure de diméthyl diallyl ammonium et d'acrylamide vendu sous la dénomination Merquat 550 par la Société Merck.

Ces polymères sont décrits plus particulièrement dans le brevet français 2 080 759 et son certificat d'addition n° 2 190 406.

(10) Le polymère de polyammonium quaternaire contenant des motifs récurrents répondant à la formule :

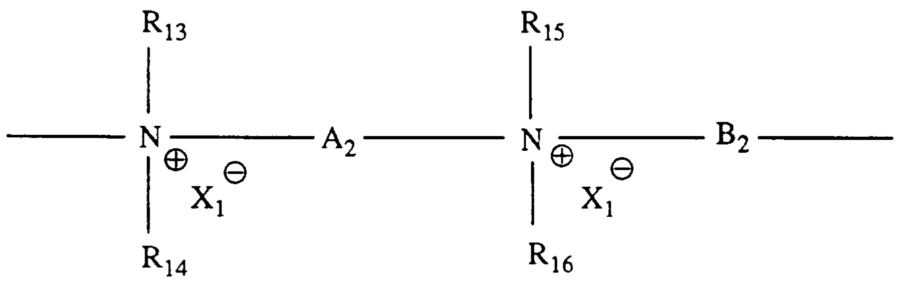

(VII)

dans laquelle $R_{13}$, $R_{14}$, $R_{15}$ et $R_{16}$,identiques ou différents, représentent des radicaux hydrocarbonés aliphatiques, alicycliques ou arylaliphatiques contenant de 1 à 20 atomes de carbone, ou des radicaux hydroxyalkylaliphatiques inférieurs ou bien les couples $R_{13}$ et $R_{14}$ et/ou $R_{15}$ et $R_{16}$ constituent, avec les atomes d'azote auxquels ils sont rattachés, des hétérocycles contenant éventuellement un second hétéroatome autre que l'azote, ou bien $R_{13}$,$R_{14}$, $R_{15}$ et $R_{16}$ représentent un radical alkyle en $C_2$-$C_6$ linéaire ou ramifié substitué par un groupement nitrile, ester, acyle, amide,

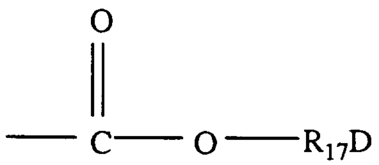

ou

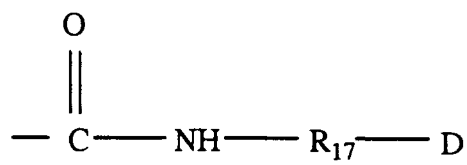

où $R_{17}$ est un alkylène et D un groupement ammonium quaternaire,

$A_2$ et $B_2$ représentent des groupements alkylène, notamment polyméthylène contenant de 2 à 20 atomes de carbone linéaires ou ramifiés, saturés ou insaturés et pouvant contenir, liés à ou intercalés dans la chaîne principale, un ou plusieurs cycles aromatiques, ou un ou plusieurs atomes d'oxygène ou de soufre, ou un ou plusieurs groupements SO, $SO_2$, disulfure, amino, alkylamino, hydroxyle, ammonium quaternaire, uréido, amide ou ester, et $X_1^{\ominus}$ désigne un anion dérivé d'un acide minéral ou organique,

$A_2$, $R_{13}$ et $R_{15}$ peuvent en outre former ensemble, avec les deux atomes d'azote auxquels ils sont rattachés, un cycle pipérazinique ; en outre, lorsque $A_2$ désigne un radical alkylène ou hydroxyalkylène linéaire ou ramifié, saturé ou insaturé, $B_2$ peut également désigner un groupement :

$$-(CH_2)_n-CO-D-OC-(CH_2)_n-$$

dans lequel D désigne :

a) un reste de glycol de formule : - O - Z - O - où Z désigne un radical hydrocarboné linéaire ou ramifié ou un groupement répondant à la formule :

$$[CH_2\text{-}CH_2\text{-}O]_x\text{-}CH_2\text{-}CH_2\text{-}$$

ou

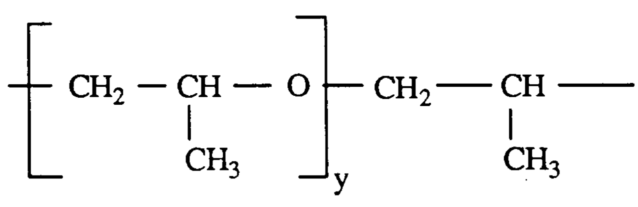

où x et y désignent un nombre de 1 à 4 représentant un degré de polymérisation défini ou moyen ;

b) un reste de diamine bis-secondaire tel qu'un reste de pipérazine :

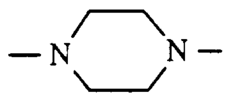

c) un reste de diamine bis-primaire de formule :

$$- NH - Y - NH -$$

où Y désigne un radical bivalent hydrocarboné linéaire ou ramifié ou bien le radical

$$- CH_2 - CH_2 - S - S - CH_2 - CH_2 -$$

d) un groupement uréylène de formule :

$$- NH - CO - NH - ;$$

$X^{\ominus}$ est un anion tel que chlorure ou bromure.

Des polymères de ce type sont décrits en particulier dans les brevets français 2.320.330, 2.270.846, 2.316.271, 2.336.434, et 2.413.907 et les brevets US-A-2.273.780, 3.206.462, 2.375.853, 2.388.614, 2.454.547, 2.261.002, 2.271.378, 3.874.870, 4.001.432, 3.929.990, 3.966.904, 4.005.193, 4.025.617, 4.025.627, 4.025.653, 4.026.945 et 4.027.020.

(11) Les polymères de polyammonium quaternaires constitué de motifs de formule :

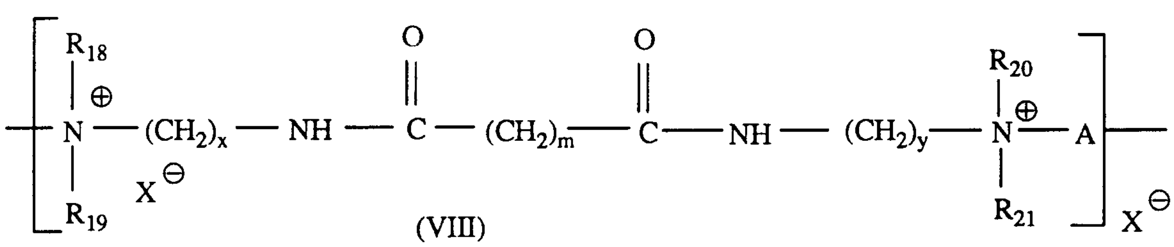

(VIII)

dans laquelle $R_{18}$, $R_{19}$, $R_{20}$ et $R_{21}$, identiques ou différents, représentent un atome d'hydrogène, ou un radical méthyle, éthyle, propyle, β-hydroxyéthyle, β-hydroxypropyle ou $-CH_2CH_2(OCH_2CH_2)_pOH$ où p est le nombre 0 ou un nombre entier compris entre 1 et 6, sous réserve que $R_{18}$, $R_{19}$, $R_{20}$ et $R_{21}$ ne représentent pas simultanément un atome d'hydrogène, x et y, identiques ou différents, sont des nombres entiers compris entre 1 et 6 ;
m est le nombre 0 ou un nombre entier compris entre 1 et 34,
X désigne un atome d'halogène,
A désigne le reste d'un radical divalent hydrocarboné comportant éventuellement des hétéroatomes, et en particulier le radical :

$$- CH_2 - CH_2 - O - CH_2 - CH_2 -$$

De tels composés sont décrits notamment dans la demande de brevet européen 122.324.

(12) Les homopolymères ou copolymères dérivés des acides acrylique ou méthacrylique et comportant des motifs :

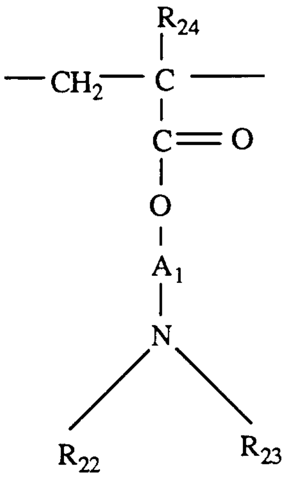 , 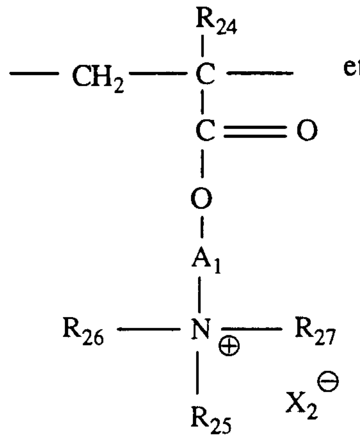 et/ou 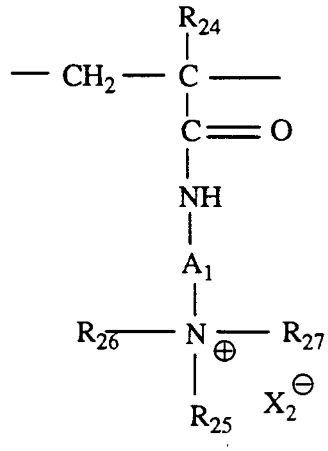

dans lesquels les groupements $R_{24}$ désignent indépendamment H ou $CH_3$,
les groupements $A_1$ désignent indépendamment un groupe alcoyle linéaire ou ramifié de 1 à 6 atomes de carbone ou un groupe hydroxyalcoyle de 1 à 4 atomes de carbone,
les groupements $R_{25}$, $R_{26}$, $R_{27}$, identiques ou différents désignant indépendamment un groupe alcoyle ayant de 1 à 18 atomes de carbone ou un radical benzyle,
$R_{22}$ et $R_{23}$ représentent hydrogène ou un groupement alcoyle ayant de 1 à 6 atomes de carbone, $X_2^-$ désigne un anion, par exemple méthosulfate ou halogénure tel que chlorure ou bromure.

Le ou les comonomères utilisables dans la préparaiton des copolymères correspondants appartiennent à la famille des acrylamides, méthacrylamides, diacétone acrylamides, acrylamides et méthacrylamides substitués à l'azote par des alcoyle inférieurs, des esters d'alcoyle des acides acrylique ou méthacrylique, la vinylpyrrolidone ou des esters vinyliques.

(13) Les polymères quaternaires de vinylpyrrolidone et de vinylimidazole tels que par exemple les produits commercialisés sous les dénominations Luviquat FC 905, FC 550 et FC 370 par la Société B.A.S.F.

D'autres polymères cationiques utilisables conformément à l'invention sont des polyalkylèneimines, en particulier des polyéthylèneimines, des polymères contenant des motifs vinylpyridine ou vinylpyridinium, des condensats de polyamines et d'épichlorhydrine, les polyuréylènes quaternaires et les dérivés de la chitine.

Parmi les polymères cationiques utilisables dans les compositions de l'invention, on citera notamment les polymères suivants ;

- le polymère comportant des motifs de formule :

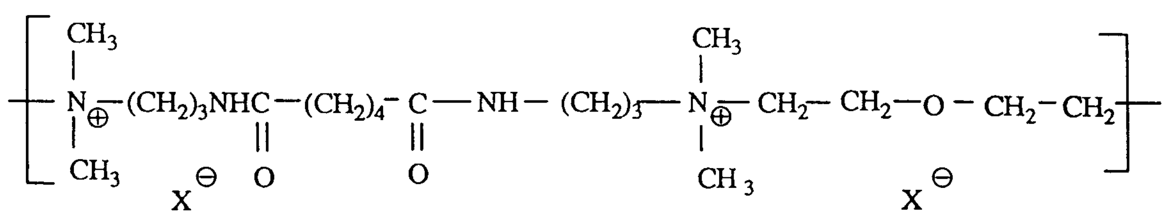

  vendu sous la dénomination "Mirapol AD 1" par la Société Miranol,
- le polymère comportant des motifs de formule :

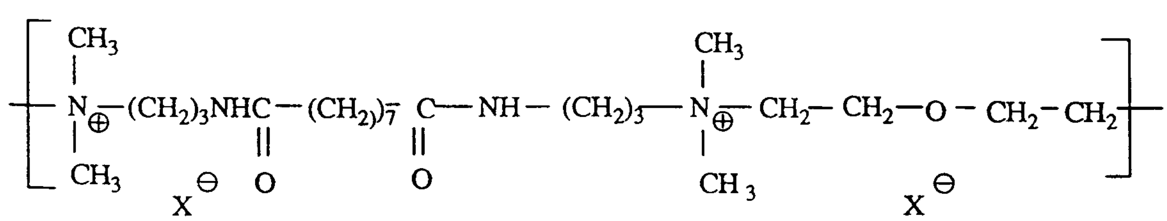

  vendu sous la dénomination "Mirapol AZ1" par la Société Miranol,

- le poly(chlorure de méthacrylamidopropyltriméthylammonium) vendu sous la dénomination "Polymaptac" par la Société Texaco Chemicals ;
- les polymères quaternisés du type ionène décrits dans le brevet français n° 2.270.846 et plus particulièrement ceux comportant les motifs :

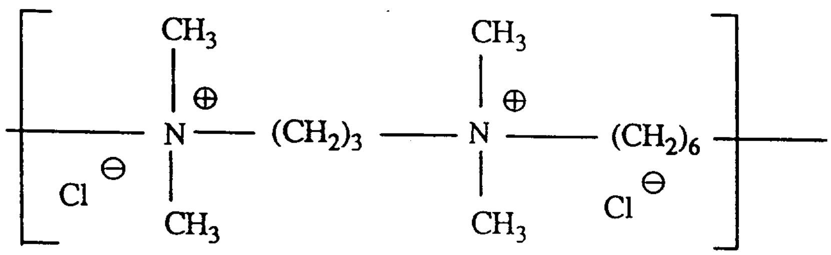

- les polymères d'ammonium quaternaires du type décrit dans le brevet US.4.157.388 et plus particulièrement celui vendu sous la dénomination "Mirapol A 15" par la Société Miranol ;
- le poly(chlorure de diméthyl butényl ammonium)-α,ω-bis-(chlorure de triéthanol ammonium) vendu sous la dénomination de "Onamer M" par la Société Onyx Internationale.

Les particules organiques destinées à être revêtues conformément à l'invention peuvent être éventuellement des particules ayant subi préalablement un ou plusieurs traitements de surface connus de nature chimique, électronique et/ou mécanique. Elles peuvent être en outre des particules minérales ou organiques revêtues d'une substance organique, telles que les particules composites qui sont décrites ci-après.

Les particules organiques destinées à être revêtues par un polymère cationique selon l'invention comprennent par exemple :

- le carmin de cochenille,
- le noir de carbone,
- les laques organiques ou sels insolubles de sodium, de potassium, de calcium, de baryum, d'aluminium, de zirconium ou de strontium de colorants acides tels que les colorants halogénoacides, azoïques, anthraquinoniques, etc... Parmi ces laques, on peut en particulier citer celles connues sous les dénominations suivantes :

| | | |
|---|---|---|
| D & C Red n° | 2 | Aluminium lake |
| D & C Red n° | 3 | Aluminium lake |
| D & C Red n° | 4 | Aluminium lake |
| D & C Red n° | 6 | Aluminium lake |
| D & C Red n° | 6 | Barium lake |
| D & C Red n° | 6 | Barium/Strontium lake |
| D & C Red n° | 6 | Strontium lake |
| D & C Red n° | 6 | Potassium lake |
| D & C Red n° | 7 | Aluminium lake |
| D & C Red n° | 7 | Barium lake |
| D & C Red n° | 7 | Calcium lake |
| D & C Red n° | 7 | Calcium/strontium lake |
| D & C Red n° | 7 | Zirconium lake |
| D & C Red n° | 8 | Sodium lake |
| D & C Red n° | 9 | Aluminium lake |
| D & C Red n° | 9 | Barium lake |
| D & C Red n° | 9 | Barium/Strontium lake |

(suite)

| | | |
|---|---|---|
| D & C Red n° | 9 | Zirconium lake |
| D & C Red n° | 10 | Sodium lake |
| D & C Red n° | 19 | Aluminium lake |
| D & C Red n° | 19 | Barium lake |
| D & C Red n° | 19 | Zirconium lake |
| D & C Red n° | 21 | Aluminium lake |
| D & C Red n° | 21 | Zirconium lake |
| D & C Red n° | 27 | Aluminium lake |
| D & C Red n° | 27 | Barium lake |
| D & C Red n° | 27 | Calcium lake |
| D & C Red n° | 27 | Zirconium lake |
| D & C Red n° | 30 | Lake |
| D & C Red n° | 31 | Calcium lake |
| D & C Red n° | 33 | Aluminium lake |
| D & C Red n° | 34 | Calcium lake |
| D & C Red n° | 36 | Lake |
| D & C Red n° | 40 | Aluminium lake |
| D & C Blue n° | 1 | Aluminium lake |
| D & C Green n° | 3 | Aluminium lake |
| D & C Orange n° | 4 | Aluminium lake |
| D & C Orange n° | 5 | Aluminium lake |
| D & C Orange n° | 5 | Zirconium lake |
| D & C Orange n° | 10 | Aluminium lake |
| D & C Orange n° | 17 | Barium lake |
| D & C Yellow n° | 5 | Aluminium lake |
| D & C Yellow n° | 5 | Zirconium lake |
| D & C Yellow n° | 6 | Aluminium lake |
| D & C Yellow n° | 7 | Zirconium lake |
| D & C Yellow n° | 10 | Aluminium Lake |

- les pigments mélaniques dérivés de sources naturelles ou synthétiques et qui peuvent être obtenus : (A) par oxydation d'au moins un composé indolique, ou (B) par polymérisation oxydante ou enzymatique de précurseurs mélaniques, ou (C) par extraction de la mélanine à partir de substances en contenant, ou (D) par culture de microorganismes.

(A) Les pigments mélaniques peuvent, en premier lieu, être obtenus par oxydation d'au moins un composé indolique choisi notamment parmi ceux répondant à la formule :

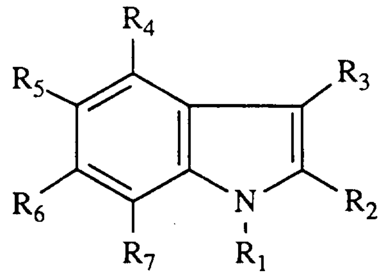

(IX)

dans laquelle :

- $R_1$ et $R_3$ représentent, indépendamment l'un de l'autre, un atome d'hydrogène ou un groupe alkyle en $C_1$-$C_4$ ;
- $R_2$ représente un atome d'hydrogène, un groupe alkyle en $C_1$-$C_4$, un groupe carboxyle ou un groupe alcoxy ($C_1$-$C_4$)-carbonyle ;
- les substituants $R_4$ à $R_7$ représentent un atome d'hydrogène, un groupement alkyle en $C_1$-$C_4$, un groupement -NHR° ou -OZ,
  R° désignant un atome d'hydrogène, un groupe acyle en $C_2$-$C_4$ ou hydroxyalkyle en $C_2$-$C_4$, et le radical Z désignant un atome d'hydrogène, un groupe acyle en $C_2$-$C_{14}$, un groupe alkyle en $C_1$-$C_4$, ou un groupe triméthylsilyle,
- étant entendu que $R_5$ peut en outre représenter un atome d'halogène,

et étant entendu que :

- au moins l'un des radicaux $R_4$ à $R_7$ représente un groupement OZ ou NHR°, l'un au plus des radicaux $R_4$ à $R_7$ représentant NHR° et deux au plus des radicaux $R_4$ à $R_7$ représentant OZ et,
  dans le cas où Z représente un atome d'hydrogène, les deux groupes OH sont dans les positions 5 et 6 ; et au moins l'un des radicaux $R_4$ à $R_7$ représente un atome d'hydrogène, et dans le cas où un seul de ces radicaux représente un atome d'hydrogène, un seul radical parmi les radicaux $R_4$ à $R_7$ représente alors NHR° ou OZ, les autres radicaux représentant un groupe alkyle en $C_1$-$C_4$, ou encore, le cas échéant, pour $R_5$, un atome d'halogène ;
  et leurs sels de métaux alcalins, alcalino-terreux, d'ammonium, d'amines, ainsi que les chlorhydrates, les bromhydrates, les sulfates et méthanesulfonates.

Les composés indoliques de formule (IX) ci-dessus sont choisis, de préférence, parmi le 4-hydroxyindole, le 5-hydroxyindole, le 6-hydroxyindole, le 7-hydroxyindole, le 4-hydroxy-5-méthoxyindole, le 4-hydroxy-5-éthoxyindole, le 2-carboxy-5-hydroxyindole, le 5-hydroxy-6-méthoxyindole,le 6-hydroxy-7-méthoxyindole, le 5-méthoxy-6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-β-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.

Le 5,6-dihydroxyindole est l'un des composés préférés.

L'oxydation du composé indolique de formule (IX)peut s'effectuer en milieu aqueux ou eau-solvant(s), à l'air, en présence ou non d'un agent alcalin et/ou d'un catalyseur métallique d'oxydation tel que par exemple l'ion cuivrique.

Le milieu réactionnel est, de préférence, constitué par de l'eau et peut, le cas échéant, être constitué par un mélange d'eau et d'au moins un solvant choisi de telle façon qu'il solubilise rapidement le composé indolique de

formule (IX). Parmi ces solvants, on peut citer, à titre d'exemples, les alcools inférieurs en $C_1$-$C_4$, tels que l'alcool éthylique, l'alcool propylique ou isopropylique, l'alcool tert-butylique, les alkylèneglycols tels que l'éthylèneglycol, le propylèneglycol, les alkyléthers d'alkylèneglycols, tels que les éthers monométhylique, monoéthylique et monobutylique de l'éthylèneglycol, les monométhyléthers du propylèneglycol et du dipropylèneglycol, et le lactate de méthyle.

L'oxydation peut également s'effectuer en mettant en oeuvre le peroxyde d'hydrogène en présence d'un agent alcalin, tel que, de préférence, l'ammoniaque, ou en présence d'un ion iodure, l'iodure étant, de préférence, l'iodure d'un métal alcalin, alcalino-terreux ou d'ammonium.

On peut également procéder à l'oxydation en utilisant l'acide periodique et ses sels hydrosolubles et dérivés, les permanganates et bichromates, par exemple de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont, notamment le cérium, et des oxydants organiques choisis parmi les ortho- et parabenzoquinones, les ortho- et parabenzoquinones mono- ou di-imines, les 1,2- et 1,4-naphtoquinones, les 1,2- et 1,4-naphtoquinones mono- ou di-imines telles que définies dans la demande EP-A-0 376 776. Le sel d'acide periodique préféré est le periodate de sodium.

Il est possible d'activer les agents oxydants par un modificateur de pH.

On peut également effectuer une oxydation enzymatique.

Le produit insoluble est isolé par filtration, centrifugation, lyophilisation ou atomisation ; il est ensuite broyé ou micronisé pour atteindre la granulométrie désirée.

(B) Les pigments mélaniques peuvent également provenir de la polymérisation oxydante ou enzymatique de précurseurs mélaniques, tels que la L-tyrosine, la L-dopa, le catéchol et leurs dérivés.

(C) les pigments mélaniques peuvent enfin provenir de l'extraction de la mélanine de substances naturelles telles que les cheveux humains ou l'encre de céphalopodes (seiches, poulpes), encore connue sous le nom de sépiomélanine, auquel cas le pigment est broyé et purifié avant son utilisation.

(D) Les pigments mélaniques peuvent être obtenus par culture de microorganismes. Ces microorganismes produisent de la mélanine soit naturellement, soit par modification génétique ou par mutagénèse. Des modes de préparations de ces mélanines sont décrits par exemple dans la demande de brevet WO-90 04029.

Le pigment mélanique peut être présent à la surface, ou incorporé dans une charge particulaire, minérale ou organique, lamellaire ou non lamellaire, colorée ou non. On obtient ainsi des pigments mélaniques composites.

Dans ce cas, le pigment mélanique peut résulter de l'oxydation d'au moins un composé indolique de formule (IX), telle que définie ci-dessus, en mélange avec la charge particulaire, dans un milieu essentiellement non-solvant pour ladite charge, à une température pouvant aller de la température ambiante jusqu'à environ 100°C, ou encore résulter de la polymérisation oxydante de précurseur mélanique sur la charge.

Les particules minérales non lamellaires utilisées dans ce procédé sont en particulier des particules minérales inertes ayant une granulométrie inférieure à 20 micromètres. De telles particules sont notamment les particules de carbonate de calcium, de silice ou d'oxyde de titane.

De tels pigments mélaniques composites, déposés sur charges minérales, sont décrits, ainsi que leur préparation, dans la demande de brevet FR-2.618.069.

Par un procédé analogue, on peut préparer des pigments mélaniques composites avec des particules minérales colorées.

On appelle "particules minérales colorées" des particules non blanches constituées de sels métalliques, insolubles dans le milieu cosmétique, utilisables en cosmétique telles que celles référencées dans le Color Index sous le chapitre "Inorganic Colouring Matters" et portant les numéros 77000 à 77947, à l'exclusion des pigments blancs et de particules se présentant sous forme lamellaire telles que l'oxyde de fer lamellaire. Ces particules minérales colorées peuvent être constituées d'un seul pigment ou d'un mélange de pigments et peuvent ainsi se présenter sous forme de pigments nacrés ou interférentiels.

Les particules minérales colorées sont en particulier des particules non blanches, choisies de préférence parmi les oxydes de fer, à l'exclusion de l'oxyde de fer lamellaire, le bleu outremer (qui est un sulfosilicate complexe), les oxydes de chrome, le violet de manganèse (qui est un pyrophosphate d'ammonium et de manganèse) et le bleu de prusse (qui est un ferricyanure de fer).

De tels pigments mélaniques composites, déposés sur charge minérale colorée, sont décrits dans la demande de brevet français 92 0415 déposée le 16 Janvier 1992.

Les particules lamellaires sont des particules minérales ou organiques se présentant sous forme de feuillets éventuellement stratifiés. Ces feuillets se caractérisent par une épaisseur plus faible que la plus grande dimension de la particule. De préférence, le rapport entre la plus grande dimension et l'épaisseur est compris entre 2 et 100. La dimension la plus grande est généralement inférieure à 50 micromètres. De tels pigments mélaniques composites déposés sur charge lamellaire sont décrits, ainsi que leur préparation, dans la demande de brevet européen n°467.767

Les particules organiques non lamellaires sont des particules de polymères inertes choisis parmi les polymères naturels ou synthétiques, organiques ou inorganiques, à réseau réticulé, cristallin ou amorphe, ayant par exemple un poids moléculaire compris entre 5 000 et 5 000 000. Des pigments mélaniques composites sur charge polymérique ainsi que leur préparation sont décrits dans la demande de brevet européen n°379 409 ;

- les particules obtenues par polymérisation oxydative d'au moins un composé indolinique de formule :

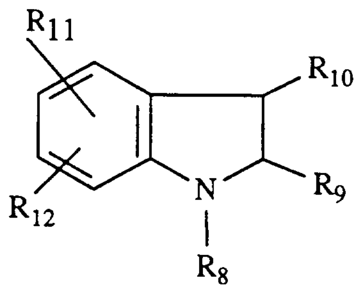

(X)

formule dans laquelle :

- $R_{10}$ et $R_8$ représentent, indépendamment l'un de l'autre, un atome d'hydrogène ou un radical alkyle en $C_1$-$C_4$ ;
- $R_9$ représente un atome d'hydrogène, un radical alkyle en $C_1$-$C_4$, un groupe carboxyle ou alcoxy ($C_1$-$C_4$) carbonyle ;
- $R_{12}$ désigne un atome d'hydrogène, un radical alkyle en $C_1$-$C_4$, hydroxyle, alcoxy ($C_1$-$C_4$), amino ou alkylamino en $C_1$-$C_{10}$, ou halogène ;
- $R_{11}$ désigne un atome d'hydrogène, un groupe hydroxyle, alcoxy en $C_1$-$C_4$, ou amino ; avec la condition qu'au moins un des radicaux $R_{11}$ ou $R_{12}$ désigne un groupe hydroxyle,alcoxy ou amino ; et avec la condition que, lorsque $R_{11}$ désigne un groupe amino, $R_{12}$ ne peut pas désigner un radical alkylamino ;
- $R_{11}$ et $R_{12}$ pouvant également former un groupement alkylènedioxy en $C_1$-$C_2$, lorsqu'ils sont en positions 5 et 6 ; et leurs sels.

Les composés répondant à la formule (X) sont choisis notamment dans le groupe constitué par la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline et la 5,6-dihydroxy 2-carboxyindoline.

Dans les composés de formule (X), les radicaux alkyle en $C_1$-$C_4$ désignent de préférence méthyle, éthyle, propyle, isopropyle, butyle, isobutyle; les radicaux en $C_1$-$C_{10}$ désignent de préférence méthyle, éthyle, propyle, isopropyle, butyle, isobutyle, 1-méthylhexyle, 1-méthylheptyle, 1-méthyloctyle ; les radicaux alcoxy désignent, de préférence, méthoxy, éthoxy, propoxy et butoxy ; halogène désigne, de préférence, brome, chlore ou iode.

Les sels des composés de formule (X) sont, en particulier, des chlorhydrates, bromhydrates, sulfates, méthanesulfonates ou des sels de métaux alcalins, alcalino-terreux, d'ammonium ou d'amines.

- Les particules obtenues par co-oxydation d'au moins un composé indolinique de formule (X) et d'au moins un dérivé d'indole. Ce dernier peut être choisi parmi les mono- et di- hydroxyindoles ou les aminoindoles, tels que décrits plus particulièrement dans le brevet EP-A-239 826 et les demandes de brevet EP-A-425 345 et GB-A-2 224 754.

Ces indoles répondent plus particulièrement à la formule (IX). Lors de la co-oxydation, on peut utiliser jusqu'à 50% en mole de dérivés indoliques par rapport au nombre total de moles de dérivés à oxyder. Les conditions d'oxydation sont identiques à celles des pigments mélaniques décrites ci-dessus.

Tout comme les pigments mélaniques, [es produits issus de la polymérisation oxydative d'au moins un composé indolinique de formule (X) peuvent être présents à la surface d'une charge particulaire, ou incorporés dans ladite

charge particulaire minérale ou organique, lamellaire ou non lamellaire, colorée ou non. Ce sont alors des pigments composites.

Les charges particulaires minérales sont celles mentionnées ci-dessus pour les pigments mélaniques composites.

Les charges organiques non lamellaires sont choisies parmi les particules de :

a) polymères dérivés de la kératine, éventuellement modifiés ;
b) fibroïnes de soie ;
c) polymères dérivés de la chitine, éventuellement désacétylés ;
d) les polymères cellulosiques ;
e) polymères synthétiques choisis parmi :

(i) le polyéthylène, le polypropylène, le polystyrène, le polyméthacrylate de méthyle éventuellement réticulé ;
(ii) la poly-β-alanine réticulée ; (iii) les polymères réticulés de styrène/divinylbenzène, de méthacrylate de méthyle/diméthacrylate d'éthylèneglycol ou de stéarate de vinyle/divinylbenzène ;
(iv) les microsphères creuses de copolymères de chlorure de vinylidène et d'acrylonitrile ;
(v) des microsphères poreuses de polyamide 12, de polyamide 6 ou de copolyamide 6/12 ;
(vi) de poudres de silicones constituées notamment par des gommes, des résines ou des élastomères d'organosiloxane.

De telles charges organiques non lamellaires sont mentionnées notamment dans la demande de brevet européen n°379 409.

Les charges lamellaires sont choisies parmi la L-lauroyllysine, les microparticules de céramique éventuellement recouvertes de poudre de zirconium, le dioxyde de titane lamellaire, le talc lamellaire, le nitrure de bore, le mica lamellaire, l'oxychlorure de bismuth, l'oxyde de fer transparent rouge.

De telles charges lamellaires sont notamment mentionnées dans la demande de brevet européen n°467 767.

De tels pigments composites ainsi que leur préparation sont notamment décrits dans la demande de brevet français n°92-00417 déposée le 16 Janvier 1992.

Dans les compositions de l'invention, les proportions des particules organiques revêtues, dispersées dans le liant, dépendent du type de composition ; il s'agit des proportions usuelles pour le type de composition considéré.

Pour enrober les particules, on peut utiliser une méthode connue, par exemple l'une des méthodes suivantes :

1) On prépare une solution du polymère dans un de ses bons solvants. On disperse dans cette solution la poudre à enrober sous agitation vigoureuse et on ajoute un mauvais solvant de polymère sans aller jusqu'à la précipitation du polymère dans la solution, mais seulement jusqu'au premier trouble. On laisse alors la suspension sous agitation vigoureuse, par exemple pendant 4 heures. On laisse décanter la suspension, on rince avec un non-solvant du polymère et on sèche, par exemple à 80°C, sous pression réduite.

2) On prépare une solution du polymère dans laquelle on disperse la poudre à enrober. On laisse le système sous agitation vigoureuse et on ajoute lentement un précipitant du polymère de façon à faire précipiter doucement le polymère à la surface de la poudre. On laisse décanter, on rince avec un non-solvant du polymère et on sèche la poudre.

3) On prépare une solution, avec un bon solvant du polymère, et on y disperse la poudre à enrober. On choisit un mauvais solvant du polymère dont le point d'ébullition est supérieur à celui du bon solvant et on réalise une évaporation lente du système. On aboutit à la formation d'un coacervat qui enrobe progressivement la poudre, puis on sèche la poudre.

4) On utilise la technique dite du lit d'air fluidisé : on pulvérise à chaud, une solution diluée du polymère dans un cyclone dans lequel la poudre est maintenue en sustentation.

5) On prépare une solution du polymère dans laquelle on disperse la poudre à enrober. On laisse le système sous agitation vigoureuse et on évapore lentement le solvant de façon à faire précipiter doucement le polymère à la surface de la poudre. On laisse décanter, on rince avec un non-solvant du polymère, et on sèche la poudre.

6) On utilise la technique d'enrobage par atomisation. On prépare une suspension de particules dans l'eau ; une fois que la suspension est homogénéisée, on y introduit une solution aqueuse du polymère. On laisse le mélange sous agitation pendant 2 heures et on atomise la suspension dans un appareil atomiseur.

Durant l'atomisation, le mélange reste, de préférence, sous agitation magnétique.

7) On utilise la technique d'enrobage par lyophilisation. On solubilise le polymère dans l'eau, puis on y incorpore sous agitation une suspension aqueuse de particules. On laisse sous agitation pendant 6 à 8 heures, puis on met le mélange sur le lyophilisateur pendant au moins 18 heures. On récupère un produit pulvérulent que l'on tamise.

Les compositions de l'invention peuvent être des compositions anhydres. Les compositions anhydres se présentent notamment sous la forme d'une poudre compactée, d'une poudre coulée, d'un rouge à lèvres ou d'un vernis à ongles.

Les compositions de l'invention peuvent également se présenter sous la forme d'émulsions du type eau-dans-l'huile ou huile-dans-l'eau et être employées par exemple comme fond de teint ou mascara.

Ces compostions sont préparées selon les méthodes usuelles.

Dans les compositions de maquillage, le liant est un liant gras usuel constitué d'une huile, d'un mélange d'huiles ou d'un mélange d'huile et de cire(s).

Dans les rouges à lèvres, le liant est également un liant gras généralement constitué par un mélange de cires de haut point de fusion (naturelles ou synthétiques), d'huiles (synthétiques, minérales ou végétales) et de cires à bas point de fusion (naturelles ou synthétiques).

Dans les vernis à ongles, le liant est constitué par la solution du polymère filmogène et du plastifiant dans le solvant organique choisi.

Dans les émulsions, le liant est un liant gras usuel constitué d'une huile ou d'un mélange d'huiles.

Lorsque la composition comporte un pigment micronisé d'oxydes métalliques choisis notamment parmi les oxydes de titane, de zinc, de cérium, de zirconium ou leurs mélanges, elle peut constituer une composition protectrice de la peau ou des cheveux contre les rayons ultra-violets.

L'invention concerne également l'utilisation,dans la préparation d'une composition cosmétique, contenant une dispersion de particules solides dans un liant, de particules organiques dont la surface est revêtue avec au moins un polymère cationique. Dans cette utilisation, la composition, et notamment les particules organiques, le polymère cationique, ainsi que le liant, sont en particulier tels que définis ci-dessus.

Les exemples suivants illustrent l'invention sans toutefois la limiter.

**EXEMPLE 1 :**

On mélange dans 200ml d'eau, 6,74g de pigment mélanique (obtenu par polymérisation oxydante du 5,6-dihydroxyindole, en présence d'eau oxygénée en milieu ammoniacal) et 0,675 g (matière active) d'un copolymère de vinylpyrrolidone et de méthacrylate de diméthylaminoéthyle quaternisé au sulfate de diméthyle, contenant 50% en poids de motifs vinylpyrrolidone ("Gafquat HS 100" de la Société GAF vendu en solution à 20% de matière active). On agite pendant 6 heures.

Le mélange est congelé dans l'azote liquide puis séché par lyophilisation. On obtient un pigment noir enrobé à 2% de polymère (% en poids par rapport au pigment mélanique).

**EXEMPLE 2 :**

On mélange dans 500ml d'eau 10g de pigment organique D & C RED n° 7 Calcium Lake et 0,9 g (matière active) de protéine de blé quaternisé ("Hydrotricum WQ" de la Société Croda vendu en solution à 28% de matière active) et on agite pendant 3 heures. On ajoute 130 ml d'acétone. Après 3 heures de contact, le pigment est récupéré par centrifugation, rincé, centrifugé à nouveau puis séché.

On obtient un pigment enrobé à 2,5% de polymère (% poids par rapport au pigment).

**EXEMPLES DE COMPOSITION**

**EXEMPLE A** : Mascara

| | |
|---|---|
| PHASE A | |
| - Acide stéarique | 8,0 g |
| - Stéarate de glycérol | 2,0 g |
| - Cire d'abeille | 7,0 g |
| - Cire de Carnauba | 2,55 g |
| - Paraffine | 10,20 g |
| - Propylparabène | 0,1 g |
| - Pigment mélanique enrobé | 1,5 g |
| PHASE B | |
| - Hydroxyéthylcellulose vendue sous la dénomination "Cellosize OP 4400 M" par la Société Amerchol | 0,5 g |
| - Méthylparabène | 0,3 g |
| - Eau | 63,95 g |
| PHASE C | |
| - Triéthanolamine | 3,9 g |

Le pigment mélanique enrobé utilisé est celui décrit à l'exemple 1.

***Mode opératoire*** :

. La phase A est fondue à 80-90°C.
. La phase C est mélangée à une partie de l'eau de la formule et ajoutée à chaud à la phase A sous vive agitation.
. Le gel B est préparé à chaud (70°C) puis ajouté à (A + C).
. Le mascara est refroidi à 25°C.

On obtient un mascara à l'aspect lisse et homogène, qui a une bonne accroche sur les cils.

**EXEMPLE B** : Brillant à lèvres

| | |
|---|---|
| - Lanoline | 23,6 g |
| - Cire de lanoline | 2,2 g |
| - Huile de vaseline | 9,30 g |
| - Cire microcristalline | 17,70 g |
| - Cire d'abeille | 4,20 g |
| - Butylhydroxytoluène | 0,15 g |
| - Béhénate d'octylglycérol | 9,20 g |
| - Huile de sésame | 12,80 g |
| - Acide oléique | 5,10 g |
| - Dioxyde de titane | 0,25 g |
| - Pigment enrobé de l'exemple 2 | 1,00 g |
| - Huile de ricin q.s.p. | 100,00 g |

On fond à 90°C les huiles et les cires constituant la phase grasse. On ajoute les pigments. Le mélange est broyé puis réchauffé à 90°C. Le mélange broyé est coulé dans des moules.

**EXEMPLE C** : Fard à joues (Blush) en crème

| | |
|---|---|
| PHASE A | |
| - Paraffine liquide | 46,7 g |
| - Vaseline | 10,0 g |
| - Cire de carnauba | 5,0 g |
| - Cire de polyéthylène | 5,0 g |
| - conservateur | 0,2 g |
| PHASE B | |
| - Oxydes de fer | 4,3 g |
| - Oxyde de titane | 8,4 g |
| - Pigment enrobé de l'exemple 2 | 0,4 g |
| PHASE C | |
| - Poudre de nylon vendue sous la dénomination "Orgasol" par la Société Atochem | 10,0 g |
| - Mica titane | 10,0 g |

***Mode opératoire*** :

La phase A est fondue à une température de 90-95°C.

On y ajoute la phase B. Le mélange peut être broyé pour une meilleure dispersion des pigments.

La phase C est ajoutée en dernier jusqu'à obtention d'une pâte homogène, puis la composition est refroidie à température ambiante.

## Revendications

1. Composition cosmétique pour la peau ou les phanères, comprenant une dispersion de particules organiques solides dans un liant, caractérisée par le fait qu'au moins une partie desdites particules organiques sont înstroduites dans ladite composition sous la forme de particules dont la surface est revêtue d'au moins un polymère cationique.

2. Composition selon la revendication 1, caractérisée par le fait que ledit polymère cationique est choisi parmi ceux comprenant des motifs qui contiennent des groupements amine primaires, secondaires, tertiaires et/ou quaternaires.

3. Composition selon la revendication 2, caractérisée par le fait que lesdits groupements amine font partie de la chaîne polymère.

4. Composition selon la revendication 2, caractérisée par le fait que lesdits groupements amine sont portés par un substituant latéral.

5. Composition selon l'une quelconque des revendications précédentes, caractérisée par le fait que lesdits polymères cationiques ont un taux de quaternisation, exprimé en équivalents cationique par gramme de polymère, d'au moins 0,05 méq cationique/g.

6. Composition selon l'une quelconque des revendications précédentes, caracterisée par le fait que lesdits polymères cationiques contiennent au moins 10% en poids de motifs comportant des groupements amine ou ammonium quaternaire.

7. Composition selon l'une quelconque des revendications précédentes, caractérisée par le fait que la proportion pondérale de polymères cationiques, par rapport au poids total des particules revêtues, est au moins égale à 0,1%.

8. Composition selon la revendication précédente, caractérisée par le fait que ladite proportion est inférieure à 10% et en particulier inférieure à 8%.

9. Composition selon l'une quelconque des revendications précédentes, caractérisée par le fait que le liant est choisi parmi les corps gras (huiles et/ou cires) et les polymères filmogènes.

10. Composition selon l'une quelconque des revendications précédentes, caractérisée par le fait qu'elle se présente sous la forme d'une poudre compactée, d'une poudre coulée, d'un rouge à lèvres, ou d'un vernis à ongles.

11. Composition selon l'une quelconque des revendications 1 à 9, caractérisée par le fait qu'elle se présente sous la forme d'une émulsion du type eau-dans-l'huile ou huile-dans-l'eau.

12. Utilisation, dans la préparation d'une composition cosmétique pour la peau ou les phanères contenant une dispersion de particules organiques solides dans un liant, de particules organiques dont la surface est revêtue avec au moins un polymère cationique.

13. Utilisation selon la revendication précédente, caractérisée par le fait que ledit polymère cationique ou ledit liant est tel que défini dans l'une quelconque des revendications 2 à 9.

## Claims

1. Cosmetic composition for the skin or superficial body growths, comprising a dispersion of solid organic particles in a binder, characterized in that at least a portion of the said organic particles are introduced into the said composition in the form of particles whose surface is coated with at least one cationic polymer.

2. Composition according to Claim 1, characterized in that the said cationic polymer is chosen from those comprising units which contain primary, secondary, tertiary and/or quaternary amine groups.

3. Composition according to Claim 2, characterized in that the said amine groups form part of the polymer chain.

4. Composition according to Claim 2, characterized in that the said amine groups are carried by a side substituent.

5. Composition according to any one of the preceding claims, characterized in that the said cationic polymers have a quaternization value, expressed as cationic equivalents per gram of polymer, of at least 0.05 cationic meq/g.

6. Composition according to any one of the preceding claims, characterized in that the said cationic polymers contain at least 10% by weight of units comprising amine or quaternary ammonium groups.

7. Composition according to any one of the preceding claims, characterized in that the proportion by weight of cationic polymers, relative to the total weight of the coated particles, is at least equal to 0.1%.

8. Composition according to the preceding claim, characterized in that the said proportion is less than 10% and in particular less than 8%.

9. Composition according to any one of the preceding claims, characterized in that the binder is chosen from fats (oils and/or waxes) and film-forming polymers.

10. Composition according to any one of the preceding claims characterized in that it is provided in the form of a compacted powder, a cast powder, a lipstick or a nail varnish.

11. Composition according to any one of Claims 1 to 9, characterized in that it is provided in the form of an emulsion of water-in-oil or oil-in-water type.

12. Use, in the preparation of a cosmetic composition for the skin or superficial body growths containing a dispersion of solid organic particles in a binder, of organic particles whose surface is coated with at least one cationic polymer.

13. Use according to the preceding claim, characterized in that the said cationic polymer or the said binder is as defined in any one of Claims 2 to 9.

## Patentansprüche

1. Kosmetische Zubereitung für die Haut oder deren Anhangsgebilde, die eine Dispersion fester organischer Teilchen in einem Bindemittel enthält, dadurch gekennzeichnet, daß mindestens ein Teil der organischen Teilchen in Form solcher Teilchen in die Zubereitung eingeführt wird, deren Oberfläche mit mindestens einem kationischen Polymer beschichtet ist.

2. Zubereitung gemäß Anspruch 1, dadurch gekennzeichnet, daß es sich bei dem kationischen Polymer um ein aus solchen Polymeren, die primäre, sekundäre, tertiäre und quaternäre Amingruppen enthaltende Einheiten umfassen, ausgewähltes Polymer handelt.

3. Zubereitung gemäß Anspruch 2, dadurch gekennzeichnet, daß die Amingruppen ein Bestandteil der Polymerkette darstellen.

4. Zubereitung gemäß Anspruch 2, dadurch gekennzeichnet, daß die Amingruppen von einem Seitenkettensubstituenten getragen werden.

5. Zubereitung gemäß einem der vorstehenden Ansprüche, dadurch gekennzeichnet, daß die Polymere ein Quaternisierungsgrad von mindestens 0,5 mÄq Kationen/g aufweisen, ausgedrückt in kationischen Äquivalenten pro Gramm Polymer.

6. Zubereitung gemäß einem der vorstehenden Ansprüche, dadurch gekennzeichnet, daß die kationischen Polymere mindestens 10 Gew.-% an Einheiten aufweisen, welche Amin- oder Ammoniumgruppen tragen.

7. Zubereitung gemäß einem der vorstehenden Ansprüche, dadurch gekennzeichnet, daß der Gewichtsanteil der kationischen Polymere, bergen auf das Gesamtgewicht der beschichteten Teilchen, mindestens 0,1 % beträgt.

8. Zubereitung gemäß dem vorstehenden Anspruch, dadurch gekennzeichnet, daß der Gewichtsanteil unter 10 %, insbesondere unter 8 % liegt.

**9.** Zubereitung gemäß einem der vorstehenden Ansprüche, dadurch gekennzeichnet, daß das Bindemittel aus Fettkörpern (Ölen und/oder Wachsen) sowie filmbildenden Polymeren ausgewählt ist.

**10.** Zubereitung gemäß einem der vorstehenden Ansprüche, dadurch gekennzeichnet, daß sie in Form eines Kompaktpuders, eines frei fließenden Puders, eines Lippenrouges oder eines Nagellackes vorliegt.

**11.** Zubereitung gemäß einem der Ansprüche 1 bis 9, dadurch gekennzeichnet, daß sie in Form einer Emulsion vom Wasser-in-Öl- oder Öl-in-Wasser-Typ vorliegt.

**12.** Verwendung von organischen Teilchen, deren Oberfläche mit mindestens einem kationischen Polymeren beschichtet ist, zur Herstellung einer kosmetischen Zubereitung für die Haut und deren Anhangsgebilde, die eine Dispersion fester organischen Teilchen in einem Bindemittel umfaßt.

**13.** Verwendung gemäß dem vorstehenden Anspruch, dadurch gekennzeichnet, daß das kationische Polymer oder das Bindemittel gemäß einem der Ansprüche 2 bis 9 definiert ist.